# EUROPEAN PATENT APPLICATION

(11) **EP 2 289 395 A1**
(43) Date of publication of application: **02.03.2011**
(21) Application number: 10182319.3
(22) Date of filing: 21.03.2006
(51) Int. Cl.: A61B 5/00, A63B 24/00, A61B 5/024, A63B 71/06

(54) **Method, mobile device and remote processing unit for training an athlete**

(62) Divisional of application: 06005732.0
(71) Applicant: Vodafone Holding GmbH, 40213 Düsseldorf (DE)
(72) Inventor: Hoeksel, Sebastiaan, 6229, VN Maastricht (NL)
(74) Representative: Müller & Schubert

(57) **Abstract**

The application relates to a method for training an athlete, wherein at least one signal indicative of the physical condition of the athlete is being sensed during the activity of the athlete and being received at a remote processing unit (2). The sensed signal is being received by the remote processing unit (2) during the activity of the athlete. Furthermore the application relates to a mobile device (1) and a remote processing unit (2) for training an athlete.

## Description

The present invention relates to a method, a mobile device and a remote processing unit for training an athlete.

With the ever growing awareness of fitness, athletes have adopted the habit of monitoring their physical condition at greater detail. Other than detecting the overall fitness of training cycles, the athletes have started to monitor their physical performance during training activities. Body signals such as the heart-rate give an indication of the capabilities of the athlete. Based on such information derived from the physical performance results, fitness programs or exercise regimes can be amended to suit the current condition of the athlete.

For the appropriate amendment of the program or regime, applications are being offered that allow the consideration of the athletes condition and the potential goals that the athlete wishes to achieve. Those applications presently present standalone solutions, where the athlete gathers the fitness data and after finishing the exercise submits the results to the application via an input device.

For the monitoring of the physical condition of the athlete systems have been suggested, wherein the heart-rate of an athlete is being measured during the exercise and being transmitted by the athlete by means of a mobile phone. For the transmission the athlete calls a service number at preset points of. Such a monitoring system is for example suggested in GB 2 375 608. With this system trends and other information relating to the heart-rate of the athlete can be made available to the athlete on a personalized web site or on the mobile phone.

The drawback of such monitoring systems is that the information obtained on the heart-rate can only be used for future exercises of the athlete.

In addition coaching systems such as videos or audio systems are known, wherein standardized exercise regimes are being presented and can be followed and carried out by an athlete watching or listening to the coaching. The drawback of those systems is that they are not adapted to the individual, present condition or needs of the athlete.

The problem to be solved by the present invention is thus to provide a training system and method wherein coaching of the athlete during his exercise can be performed and the coaching can be personalized.

According to a first aspect of the invention, the problem is solved by a method for training an athlete, wherein at least one signal indicative of the physical condition of the athlete is being sensed during the activity of the athlete and being received by a remote processing unit. The message is characterised in that the sensed signal is being received during the activity of the athletes. By receiving sensed signals during the activity of the athlete, the inventive method allows for processing of the received signal while the athlete is still performing his exercise.

Preferably the signals indicative of the physical condition of the athlete are measured continuously during the exercise. With a transmission of this continuously measured physical condition the remote processes unit can offer a real-time processing of the received signals.

According to one embodiment of the present invention the signal received by the remote processing unit is being processed to generate health data. As the mobile device in this embodiment only serves as a transmitter and potentially as a sensor, it can be comparatively small and of simple technology. In particular no calculation power and only a limited storage or memory is required on the mobile device. The remote processing unit in contrast is not limited and thus a fast processing of the sensed and received signals can be ensured. The health data which can be derived from the signal received at the remote processing unit can for example be the heart rate of the athlete.

Preferably the signal and / or the health data is being compared to information stored in the remote processing unit. The stored information can be personalized information of the athlete. By comparing the received signal or health data to such stored information, appropriate adaptations of the exercise presently performed by the athletes can be realized. The personalized information of the athletes which is stored in the remote processing unit is preferably stored in a user profile. Thereby several athletes can access the same remote processing unit and sensed signals or health data will only be compared to respective information stored for the individual athlete.

Besides health data such as heart rates the personalized information which is stored in the remote processing unit can further contain coaching information. By providing coaching information on the remote processing unit, rather than on the mobile device, where processing power is limited, a more detailed and adapted coaching can be realized. In addition the coaching information can be supplemented with other personal information stored on the remote processing unit. Such supplemental personalized information can for example be previous test results or declared goals of the athlete.

Coaching information according to the present invention can comprise indications of changes in the exercise presently performed. The provision of such coaching information is only possible with the inventive method, as the current health data or sensed signals are received at the remote processing unit in real-time.

Preferably at least one instruction signal is being generated based on the health data and/or the comparison with stored information and is being transmitted to a mobile device of the athletes. By comparing data with stored information deviations to a planed exercise regime or overexertion can be easily detected. As the sensed signals or health data is being received in real-time, instruction signals indicating necessary changes to the exercise can be transmitted in real-time as well and the training results can thus be optimized.

According to the present invention it is possible, that the instruction signal is being prioritised and the mode of presentation of the signal at the mobile device is being selected dependent of the priority of the signal. By prioritising the instruction signals, preferably at the remote processing unit, it can be ensured, that crucial instruction signals will be brought to the athlete's attention before merely indicative or advisory information is being made available to the athletes. As the instruction signal is being transferred in real-time to the mobile device, the priority of instruction signals is preferably indicated to the athletes by means of different presentation modes. Possible modes of presentation comprise audio, video and /or haptic display. These different modes of presentation ensure recognition of instruction signals and their priority by the athletes.

In addition to health data or sensed signals indicative of physical conditions, location related data can be obtained at the mobile device and transmitted to the remote processing unit. The location related data can for example be obtained by means of positioning systems such as GPS. As the remote processing unit receives the location related data, this information can be processed together with the received health data, sensed signals and/or additional information stored at the remote processing unit. With location related data being available for the current position of the athlete at the remote processing unit, circumstances of the present surrounding of the athletes can be considered for coaching the athlete. Thereby the training results can be improved.

Location related data which is obtained by the mobile device can for example be position data and/or velocity data. If the speed of the athlete is too high compared to stored training information an instruction signal can be generated to instruct the athlete to decrease the speed. Also if for example the heart beat of the athlete is too high, the present velocity of the athlete can be checked for potential decrease to lower the heart rate.

The location data can preferably be compared to geographic information stored at the remote processing unit. Geographic information in this respect can for example be a map of the present surrounding of the athlete. By comparing the location related data and in particular the position of the athlete to map information available at the remote processing unit, an optimal route according to the present physical condition of the athlete can be determined and suggested to the athlete.

According to a second aspect of the invention a mobile device for training an athlete is provided. The mobile device comprises a sensing unit for receiving a sensed signal indicative of the physical condition of the athlete and a transmission unit for transmitting the sensed signal to a remote processing unit. The sensing unit of the inventive mobile device can serve for the actual sensing of signals indicative of the physical condition of the athlete or can be in communication with a separate sensor. In the latter case the sensing unit receives the sensed signals from the sensor device.

By providing a sensing unit and a transmission unit in the mobile device a continuous transmission or a transmission at preset times can be automated. There is hence no need for the athlete to interrupt his exercise to manually transmit the sensed signals, as is the case in the prior art. The transmission of the sensed signals according to the present invention can be performed via a communication network, such as a mobile network and/or the Internet. No adaptation of the mobile device for a specific transmission method is hence necessary.

Preferably the mobile device comprises a receiving unit for receiving instruction signals from a remote processing unit. The provision of a receiving unit for instruction signals enables real time coaching via the mobile device. The coaching instructions can be generated at the remote processing unit, so that the processing power necessary at the mobile device is low.

In addition, the mobile device can comprise a processing unit for processing the received instruction signals. The processing within the processing unit can be the storing of received instruction signals and/or forwarding of the instruction signals to different units within the mobile device.

In particular the received instruction signals can be forwarded to at least one presentation unit. According to one embodiment the mobile device comprises at least two presentation units connected to the receiving unit and/or the processing unit. The benefit of providing two presentation units, which preferably provide different presentation modes, lies in the opportunity to appropriately bring the instruction signals to the athlete's attention. As the presentation units are connected to the receiving unit and/or the processing unit, the presentation mode suitable for the respective instruction signal can be used. The presentation mode is preferably selected according to the priority of the instruction signal. The correlation of priority and presentation mode can be set the remote processing unit. It is, however, also possible to set this correlation within the processing unit of the mobile device. Dependent on the priority and the related presentation mode, the respective presentation unit will receive the instruction signal for presentation.

In a preferred embodiment the mobile device further comprises means for obtaining location related data and means for transmitting the location related data to a remote processing unit. The means for obtaining location related data can for example be a GPS-receiver. The means for obtaining the location related data may be integral within the mobile device, but can also be in communication connection to the mobile device. The location related data is preferably only received and transmitted by the mobile device, i.e. no processing of location related data is being performed on the mobile device. Thereby the calculation power necessary within the mobile device can be kept to a minimum.

According to another aspect of the invention a remote processing unit for training an athlete is provided. The remote processing unit comprises a storage unit with a user interface for access to the storage unit from an input device. By providing an input device parameters within a user profile, which may be stored in the storage unit, can be set. The provision of an interface for a separate input device in addition to the mobile device of the athlete provides for easier handling of parameters. The input device can for example be a computer connected to the remote processing unit via the Internet. The provision of the interface for an input device also allows third parties, such as the coach of the athlete to update or amend the user profile.

The storage unit comprises preferably at least two user profiles. By storing the information in user profiles several athletes can access the remote processing unit and utilize the processing unit during their exercise.

According to a preferred embodiment the remote processing unit comprises an instruction signal creation unit connected to a comparison unit and to the storage unit. Via the connection to the comparison unit the instruction signal creation unit can obtain information on deviations of health data to an exercise regime. Also geographical information can be obtained from the comparison unit. With this connection suitable instruction signals can be generated. In addition, the instruction signal creation unit is connected to the storage unit of the remote processing unit. In the storage unit personalized information, preferably in form of user profiles, is being stored. With the connection to the storage unit for example priority settings with respect to a presentation mode of the instruction signals can be obtained. Advantages and features described with respect to the inventive message also apply to the inventive mobile device and the inventive remote processing unit, where applicable, and vice versa.

The present invention will now be described in detail with reference to the attached figures, wherein:
Figure 1 shows a schematic structure of a system to be used for the inventive method and
Figure 2 shows a schematic view of the structure of an embodiment of a mobile device.

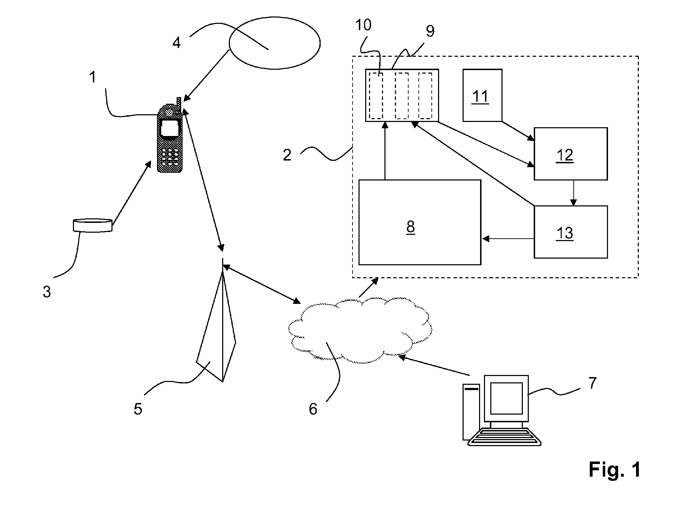

In Figure 1 the system comprises a mobile device 1 represented by a cellular phone as well as a remote processing unit 2. The mobile device 1 is connected to a sensor 3, which for example can be attached to the athlete's arm or leg. The connection between the sensor 3 and the mobile device 1 maybe wireless or by means of an appropriate plug provided at the mobile device 1 for cable connection. Furthermore Figure 1 schematically shows a positioning system 4 which uses the mobile device 1 as a receiver for location related data. The communication connection between the mobile device 1 and the remote processing unit 2 is represented by a mobile network and the Internet 6. The mobile network is indicated by means of a single base station 5.

Also an input device 7, which is indicated as a computer, is shown to be connected to the remote processing unit 2 via the Internet 6.

The structure of the remote processing unit 2 comprises in the depicted embodiment five units 8, 9, 11, 12 and 13. These units maybe realised as separate physical units or may partially be combined. In particular the units maybe realised as programs. The receiving unit 8 is provided for receiving signals and data from the mobile device 1 via the mobile network and the Internet 6. In addition to the receiving unit 8 a storage unit 9 is provided. Within the storage unit user profiles 10 are being stored. Furthermore a storage unit for geographic data 11 is provided within the remote processing unit 2. The storage unit for geographical data 11 can be combined with the storage unit 9. The remote processing unit 2 further comprises a comparison unit 12 as well as an instruction signal creation unit 13.

With a system as shown in Figure 1 an athlete can conduct his training as follows. Before starting his exercise the athlete will attach the sensor 3 to his body, so that bio data, such as the pulse rate, can be sensed during the exercise. Once the athlete has connected the sensor 3 to the mobile device 1 and has initiated the receiving and transmission on the mobile device 1, he can begin his exercise. During the exercise the sensed signals from the sensor 3 will be transmitted to the mobile device 1. The mobile device 1 will continuously forward the sensed signals to the remote processing unit 2, in particular to the receiving unit 8. The received signal maybe processed within the remote processing unit 2 to generate health data, if necessary. The sensed signal and/or the generated health data will be forwarded to the comparison unit 12 which is connected to at least the storage unit 9.

Via the connection between the storage unit 9 and the comparison unit 12, personalized information relating to the athlete can be retrieved from the storage unit. In particular personalized information from a user profile 10 for this specific athlete will be provided to comparison unit 12. By comparing the received sensed signals and/or health data to entry's in the user profile 10 deviations from normal conditions or presets exercising regimes can be determent. The comparison result maybe forwarded to an instruction signal creation unit 13. This instruction signal creation unit 13 may also be connected to the storage unit 9. Via the connection to the storage unit 9 the instruction signal creation unit 13 may obtain information for the correlation of the priority of an instruction signal to a presentation mode for mobile device 1 of the respective athlete. The thus generated instruction signal possibly together with priority information or presentation mode information can be forwarded to the receiving unit 8, which at that point can serve as a transmission unit. From the receiving/transmission unit 8 the instruction signal can be transferred to the mobile device 1 and be presented to the athlete.

If the mobile device 1 is adapted to receive signals from a positioning system 4, this information can be used as follows. The mobile device 1 receives location related data from the positioning system 4 and transmits this data to the remote processing unit 2. The receiving unit 8 may forward this data to the comparison unit 12 which is also connected to the storage unit for geographical information 11. By comparing the current position of the athlete to stored geographical information, such as maps, the comparison unit may determine a possible route for the athlete. Such a determination of a route will preferably be conducted by considering information obtainable from the user profile. The geographical information stored in storage 11 may also include information on steepness or length of potential routes for the athlete to choose from.

The comparison unit 12 will identify the appropriate route according to the information from the user profile 10 and the geographical information 11. In a further preferred embodiment the comparison unit 12 will also consider any available sensed signals or health data from the athlete to determent the appropriate route. The comparison results, i.e. a specific route or direction to or on a route will be forwarded to the receiving unit 8 which again will serve as a transmission unit. The thus created direction information will be forwarded to the mobile device 1 and presented to the athlete. The presentation may for example be via display of mobile device 1.

To initiate the ability for using the remote processing unit with the mobile device 1 the athlete may access the remote processing unit 2 via his mobile device 1 or via an input device, such as a computer 7. Via the input device 7 or the mobile device 1 the athlete can register for the training service and may also update or amend his user profile. In addition, the athlete may download records concerning his health data from the remote processing unit 2. Those records may be created during the exercise. If such records are to be created the sensed signals or health data will be transferred from the receiving unit 8 to the storage unit 9 to be included in the user profile 10 of the respective athlete.

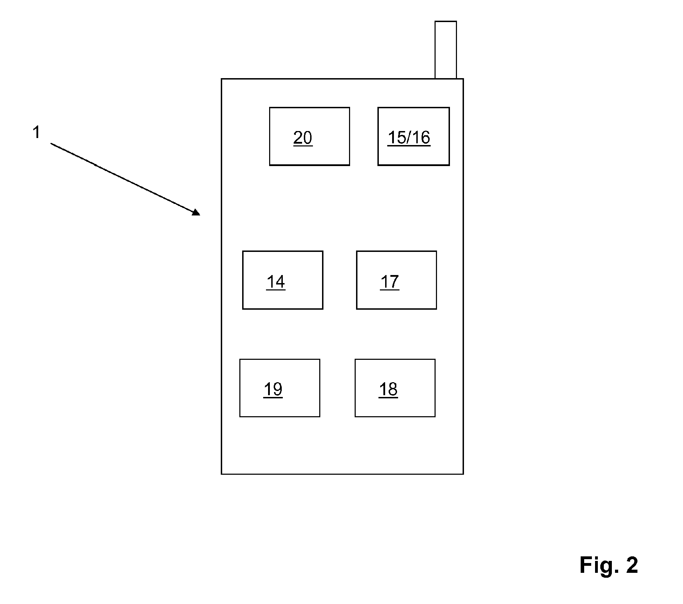

Figure 2 depicts a schematic structure of one embodiment of a mobile device 1 according to the present invention. The mobile device 1 comprises a transmission unit 15 which is integral with a receiving unit 16 in Figure 2. Further more the mobile device 1 comprises a processing unit 17, presentation units 18, 19, wherein 18 may be a loudspeaker and 19 a display. Finally a mobile device according to Figure 2 comprises a GPS-receiver unit 20.

The present invention is not limited to the embodiment shown in the figures. In particular one or more of the depicted units maybe omitted or combined with other units. Also the sensor 3 may be integral within the mobile device 1.

The present invention, hence, provides the possibility of continuously measuring relevant parameters which describe the athlete's physical performance, for example his speeds, heart rate and geographic location. These parameters can be sent by the mobile network connection to the remote processing unit which may be a network server. The remote processing unit can continuously determine the optimal coaching information, such as speed, track or route, taking into account the user profile data and the measured parameters. The present invention, thus, ensure that coaching information can be calculated in the network and takes optimal advantage of user profile data stored in the network. All real-time measurements of the athletes are available and can be considered when determining coaching information. In addition network based execution environments have a much higher processing speed than a typical client sport device and can thus calculate more intelligent coaching information.

## Claims

1. Method for training an athlete, wherein at least one signal indicative of the physical condition of the athlete is being sensed during the activity of the athlete and being received at a remote processing unit (2), **characterized in that** the sensed signal is being received by the remote processing unit (2) during the activity of the athlete.

2. Method according to claim 1, **characterized in that** the signal is being processed at the remote processing unit (2) to generate health data.

3. Method according to claim 1 or 2, **characterized in that** the signal and/or the health data is being compared to information stored in the remote processing unit (2), wherein the stored information is personalized information of the athlete.

4. Method according to any of claims 1 to 3, **characterized in that** the personalized information is stored in a user profile (10).

5. Method according to claims 3 or 4, **characterized in that** the personalized information contains coaching information.

6. Method according to any of claim 3 to 5, **characterized in that** based on the health data and/or the comparison with stored information, at least one instruction signal is being generated and transmitted to a mobile device (1) of the athlete.

7. Method according to claim 7, **characterized in that** the instruction signal is being prioritized and the mode of presentation of the signal at the mobile device (1) is being selected dependent of the priority of the signal, wherein the modes of presentation optionally comprise audio, video and/or haptic display.

8. Method according to any of claims 1 to 7, **characterized in that** the mobile device (1) obtains location related data and the location data is received at the remote processing unit (2).

9. Method according to claim 8, **characterized in that** the location related data is position data and/or velocity data and/or the location related data is being compared to geographic information stored at the remote processing unit (2).

10. Mobile device for training an athlete comprising a sensing unit (14) for receiving a sensed signal indicative of the physical condition of the athlete and a transmission unit (15) for transmitting the sensed signal to a remote processing unit (2).

11. Mobile device according to claim 10, **characterized in that** the mobile device (1) comprises a receiving unit (16) for receiving instruction signals from a remote processing unit (2).

12. Mobile device according to claim 11, **characterized in that** the mobile device (1) comprises a processing unit (17) for processing the instruction signals and the mobile device (1) optionally comprises at least two presentation units (18, 19) connected to the receiving unit (16) and / or the processing unit (17).

13. Mobile device according to any of claims 10 to 12, **characterized in that** the mobile device (1) comprises means for obtaining location data (20) and means for transmitting location data (15) to a remote processing unit (2).

14. Remote processing unit for training an athlete comprising a storage unit (9, 11) with a user interface for access to the storage unit (9, 11) from an input device (7).

15. Remote processing unit according to claim 14, **characterized in that** the storage unit (9) comprises at least two user profiles (10).

16. Remote processing unit according to claims 14 or 15, **characterized in that** the remote processing unit (2) comprises an instruction signal creation unit (13) connected to a comparison unit (12) and to the storage unit (9).
